# EUROPÄISCHE PATENTSCHRIFT

(11) **EP 2 882 469 B1**
(45) Veröffentlichungstag und Bekanntmachung des Hinweises auf die Patenterteilung: **20.09.2017**
(21) Anmeldenummer: 13737205.8
(22) Anmeldetag: 10.07.2013
(51) Int. Cl.: A61M 1/00

(54) **ANSCHLUSSVORRICHTUNG ZUR VERWENDUNG BEI DER UNTERDRUCKTHERAPIE VON WUNDEN**
CONNECTION DEVICE FOR USE IN THE NEGATIVE PRESSURE THERAPY OF WOUNDS
DISPOSITIF DE RACCORDEMENT DESTINÉ À ÊTRE UTILISÉ DANS LE TRAITEMENT DE PLAIES PAR UNE PRESSION NÉGATIVE

(30) Priorität: 09.08.2012 DE 102012214182
(43) Veröffentlichungstag der Anmeldung: 17.06.2015
(73) Patentinhaber: Paul Hartmann Aktiengesellschaft, 89522 Heidenheim (DE)
(72) Erfinder: ECKSTEIN, Axel, 89522 Heidenheim (DE); CROIZAT, Pierre, 89542 Herbrechtingen (DE); WOLF, Cornelia, 89542 Herbrechtingen (DE)
(74) Vertreter: DREISS Patentanwälte PartG mbB
(86) Internationale Anmeldenummer: PCT/EP2013/064528
(87) Internationale Veröffentlichungsnummer: WO 2014/023503

(56) Entgegenhaltungen:
- EP-A2- 1 129 734
- DE-A1-102010 006 272
- US-A1- 2006 100 586
- US-A1- 2010 063 464
- US-A1- 2011 152 800
- US-A1- 2012 143 156

## Beschreibung

Die Erfindung betrifft eine Anschlussvorrichtung zur Verwendung bei der Unterdrucktherapie von Wunden (siehe z.B. US 2006/0100586 A1), mit einem mit Unterdruck und/oder fluiden Medien beaufschlagbaren biegsamen Leitungsmittel mit wenigstens zwei Lumen und mit einem mit dem Leitungsmittel verbundenen Kopplungskörper, wobei der Kopplungskörper auf einen die Wunde überfangenden und gegen die Atmosphäre dichtend abschließenden Unterdruckverband aufbringbar ist, wobei das Leitungsmittel durch wenigstens eine Öffnung typischerweise in einer dem Unterdruckverband zugewandten Wandung des Kopplungskörpers und durch wenigstens eine Öffnung in dem Unterdruckverband hindurch mit dem Wundraum kommuniziert.

In der jüngeren Vergangenheit hat die Unterdruckbehandlung von Wunden, insbesondere von problematisch heilenden Wunden, eine zunehmende Bedeutung erlangt. Dabei bedeutet Unterdruckbehandlung, dass ein an sich der umgebenden Atmosphäre ausgesetzter Körper- oder Wundbereich durch noch näher zu beschreibende Mittel druckdicht bzw. unterdruckdicht gegen die Umgebung, also die Atmosphäre in der wir leben und atmen, abgeschlossen wird, wobei innerhalb des abgeschlossenen Wundbereichs auf ebenfalls noch zu erläuternde Weise ein gegenüber dem Atmosphärendruck verringerter Druck, mithin also Unterdruck relativ zur Atmosphäre angelegt und dauerhaft aufrechterhalten werden kann. Wenn auf dem hier in Rede stehenden Gebiet von Unterdruck die Rede ist, so wird hiermit ein Druckbereich verstanden, der typischerweise zwischen 0 und 250 mmHg (mm Quecksilbersäule) unterhalb des umgebenden Atmosphärendrucks liegt. Es hat sich gezeigt, dass dies der Wundheilung förderlich ist. Für den unterdruckdichten Abschluss wird ein Unterdruckverband vorgesehen, der beispielsweise eine druck- bzw. unterdruckdichte Folienschicht umfassen kann, die typischerweise mit einem die Wunde umgebenden unversehrten Körperbereich verklebt ist, so dass auf diese Weise ein dichter Abschluss erreicht werden kann. Um ausgehend von einer Unterdruck erzeugenden Einrichtung, also einer Unterdruckpumpe im weitesten Sinn, einen Unterdruck an den Wundraum heranzuführen und dort aufrechtzuerhalten, können bei hier in Rede stehenden Systemen zur Unterdrucktherapie von Wunden mit Unterdruck beaufschlagbare Leitungsmittel verwendet werden, die mittels einer Anschlussvorrichtung mit dem Unterdruckverband zusammenwirken, um Unterdruck an bzw. in den Wundraum zu bringen.

Aus der nicht veröffentlichten DE 10 2011 082 341.7 ist eine gattungsgemäße Anschlussvorrichtung bekannt, bei der sich zur Durchführung einer sogenannten Instilltherapie eine Fluidzuführleitung durch den Kopplungskörper hindurch erstreckt, um in den Wundraum einzuragen, so dass mit Wirkstoffen versetzte fluide Medien in den Wundraum eingeleitet werden können. Die Hindurchführung der Fluidzuführleitung durch den Kopplungskörper ist mit aufwändigen Herstellungsmaßnahmen verbunden.

Der vorliegenden Erfindung liegt die Aufgabe zugrunde, eine Anschlussvorrichtung der eingangs genannten Art dahingehend zu verbessern, dass sie sich bei Ausführung einer Unterdrucktherapie von Wunden zugleich zur Einleitung von insbesondere mit Wirkstoffen versetzten fluiden Medien in den Wundraum eignet. Die Anschlussvorrichtung soll dabei auf einfache und wirtschaftliche Weise herstellbar sein und sich im Gebrauch als betriebssicher erweisen.

Die Erfindung ist definiert durch Anspruch 1. Die Aufgabe wird bei einer Anschlussvorrichtung der genannten Art erfindungsgemäß dadurch gelöst, dass der Kopplungskörper aus einem elastomeren biegsamen Material ausgebildet ist, welches unlösbar an einen wundseitigen Endabschnitt des Leitungsmittels angespritzt ist, dass an dem Kopplungskörper ein Fluideinleitungsabschnitt ausgebildet ist, der über die dem Unterdruckverband zugewandte Wandung des Kopplungskörpers vorsteht und durch die Öffnung in dem Unterdruckverband hindurch in den Wundraum oder in eine dort vorgesehene Wundeinlage einragen kann, wobei dieser Fluideinleitungsabschnitt über das Innere des Kopplungskörpers mit einem Lumen zum Zuführen eines fluiden Mediums strömungsverbunden ist, welches Lumen entweder innerhalb des Leitungsmittels ausgebildet ist oder von einer zusätzlichen an den Kopplungskörper herangeführten Fluidzuführleitung gebildet ist.

Über das Lumen zum Zuführen eines fluiden Mediums sollen wundheilungsfördernde Fluide und/oder Wirkstoffe, die in dem fluiden Medium enthalten sind, bestimmungsgemäß dem Wundraum zugeführt werden (Instilltherapie). Über wenigstens ein weiteres als Sauglumen ausgebildetes Lumen des Leitungsmittels wird das fluide Medium und gegebenenfalls zusätzliche Wundflüssigkeit aus dem Wundraum abgesaugt und in Richtung auf die Unterdruck erzeugende Einrichtung in einen Flüssigkeitssammelbehälter gefördert.

Das vorzugsweise flach bauende Leitungsmittel der erfindungsgemäßen Anschlussvorrichtung wird zunächst separat hergestellt, was den grundsätzlichen Vorteil der Möglichkeit einer endlosen Fertigung, vorzugsweise durch Extrusion, mit sich bringt, wobei im Anschluss daran Abschnitte einer jeweils gewünschten Leitungslänge durch Ablängung bereitgestellt werden können. Der Kopplungskörper wird dann durch Anspritzen an einen Endabschnitt des Leitungsmittels gebildet und durch Anfügen und Befestigen der Abschlusskappe komplettiert. Es erweist sich als besonders vorteilhaft, dass bei dieser Art der Herstellung des Kopplungskörpers zugleich ein dichtender Übergang zu dem Leitungsmittel hin prozesssicher ausgebildet werden kann, oder anders ausgedrückt, dass im Zuge der Bildung des Kopplungskörpers zugleich das zugehörige und bereitgestellte Leitungsmittel dichtend an den Kopplungskörper angefügt wird.

Es sei auch erwähnt, dass die wenigstens eine Öffnung in der dem Unterdruckverband zugewandten Wandung des Kopplungskörpers entweder im Zuge des Spritzgießvorgangs durch geeignete Ausbildung des Spritzgießwerkzeugs hergestellt werden kann oder in einem nachfolgenden Arbeitsgang, beispielsweise durch einen Stanzvorgang, ausgebildet werden kann. Der Herstellung im Zuge des Spritzgießvorgangs wird indessen der Vorzug gegeben. Vorzugsweise werden mehrere Öffnungen vorgesehen, die vorzugsweise langlochförmig ausgebildet sind.

Es erweist sich als vorteilhaft, wenn das Leitungsmittel im Wesentlichen parallel zu der Ebene der flächenhaften Erstreckung des Kopplungskörpers an den Kopplungskörper herangeführt ist. In diesem Zusammenhang wird unter "im Wesentlichen parallel" eine Neigung zu dieser Erstreckungsebene, die auch die Anlagefläche an den Unterdruckverband bildet, von bis zu 15° verstanden.

Der von dem Kopplungskörper vorstehende Fluideinleitungsabschnitt kann auf verschiedene Weise ausgebildet werden. Es kann sich beispielsweise als vorteilhaft erweisen, wenn der Fluideinleitungsabschnitt von der an den Kopplungskörper herangeführten Fluidzuführleitung gebildet ist, die sich durch den Kopplungskörper hindurcherstreckt, wobei das den Kopplungskörper bildende elastomere biegsame Material auch an die Fluidzuführleitung angespritzt sein kann. Solchenfalls wird also die Fluidzuführleitung zusammen mit dem Leitungsmittel in ein Spritzgusswerkzeug eingelegt und ebenso wie das Leitungsmittel durch Anspritzen von elastomerem Material in den Kopplungskörper integriert. Auch wenn keine zusätzliche Fluidzuführleitung vorgesehen ist, sondern das Leitungsmittel zugleich das Lumen zum Zuführen des fluiden Mediums bildet wäre es denkbar, dass sich das Leitungsmittel mit einem dieses Lumen begrenzenden Abschnitt durch den Kopplungskörper hindurcherstreckt und von dessen wundzugewandter Seite vorsteht.

Nach einer weiteren Variante der vorliegenden Erfindung kann es sich als vorteilhaft erweisen, wenn der Fluideinleitungsabschnitt einstückig mit dem Kopplungskörper im Spritzgießverfahren ausgebildet ist. Bei dieser Variante erweist es sich als vorteilhaft, dass die Fluidzuführleitung oder auch das Leitungsmittel nicht von ihrer im Wesentlichen parallel zur Körperoberfläche herangeführten Erstreckung abgeknickt zu werden braucht.

Nach einer weiteren Alternative kann es sich als vorteilhaft erweisen, wenn der Fluideinleitungsabschnitt als von dem Kopplungskörper separat hergestelltes Leitungsbauteil ausgebildet ist, das in eine mit dem Inneren des Kopplungskörpers strömungsverbundene Öffnung in der dem Unterdruckverband zugewandten Wandung des Kopplungskörpers vorzugsweise dichtend eingesetzt und darin fixiert ist. Diese Variante erweist sich insoweit als vorteilhaft, als die Komplexität des hierfür benötigten Spritzgießwerkzeugs begrenzt bleibt. Es muss lediglich eine mit dem Inneren des Kopplungskörpers strömungsverbundene Öffnung ausgebildet werden, in die dann der Fluideinleitungsabschnitt, einzusetzen ist. Der Fluideinleitungsabschnitt kann dabei lediglich klemmschlüssig oder unter zusätzlicher Verwendung eines Klebers fixiert sein.

Es kann sich weiter als vorteilhaft erweisen, wenn zur weiteren Ausbildung des Kopplungskörpers an das angespritzte Material eine Abschlusskappe angefügt und vorzugsweise unlösbar befestigt ist, wodurch der Kopplungskörper nach außen dicht abgeschlossen ist. Dies bringt den Vorteil mit sich, dass sich die Ausbildung des Spritzgießwerkzeugs weniger komplex gestaltet und die Ausbildung von mit den Lumen des Leitungsmittels kommunizierenden Hohlräumen innerhalb des Kopplungskörpers, die ebenfalls als Lumen bezeichnet werden, herstellungstechnisch einfacher ist. Der Kopplungskörper kann dabei nach dem Spritzvorgang nach außen offen ausmündet und wird dann durch die Abschlusskappe verschlossen und komplettiert. Die Abschlusskappe wird dabei vorzugsweise mittels Kleber dichtend fixiert.

Nach einem an sich selbständig schutzbegründenden Erfindungsgedanken ist die zusätzliche an den Kopplungskörper herangeführte Fluidzuführleitung durch diese Abschlusskappe hindurch mit dem Inneren des Kopplungskörpers strömungsverbunden. Die Abschlusskappe bildet dabei denjenigen Teil der Anschlussvorrichtung über den oder mittels dessen eine Fluidzuführung zu der Anschlussvorrichtung für die Ausführung einer Instilltherapie erfolgt. Dies bringt den Vorteil mit sich, dass verschieden ausgebildete Abschlusskappen hergestellt und vorgehalten werden können, so dass ausgehend von einer hergestellten Einheit aus Leitungsmittel mit angespritztem Kopplungskörper Anschlussvorrichtungen hergestellt werden können, die sich für die Durchführung einer Instilltherapie eignen und solche, die sich nicht für die Durchführung einer Instilltherapie eignen, je nachdem welche Art von Abschlusskappe zur Komplettierung des Kopplungskörpers verwendet wird.

In Weiterbildung des vorbeschriebenen Erfindungsgedankens erweist sich als vorteilhaft, wenn die Abschlusskappe eine Anschlusseinrichtung für die zusätzliche an den Kopplungskörper herangeführte Fluidzuführleitung aufweist. Die Fluidzuführleitung soll also in irgendeiner geeigneten Weise, etwa durch eine im einfachsten Fall klemmschlüssig wirkende Steckverbindung, mit der Abschlusskappe strömungsmäßig verbunden werden können. Solchenfalls weist die Abschlusskappe selbst eine Durchgangsöffnung in das Innere des Kopplungskörpers auf. Es besteht die Möglichkeit, dass die Fluidzuführleitung innerhalb der Abschlusskappe endet oder sich durch die Abschlusskappe hindurch erstreckt und in das Innere des Kopplungskörpers einragt oder weiter über die wundzugewandte Seite des Kopplungskörpers zur gleichzeitigen Bildung des Fluideinleitungsabschnitts vorsteht.

Im Inneren des Kopplungskörpers sind vorzugsweise wenigstens zwei jeweils unabhängig voneinander begrenzte Lumen ausgebildet, die mit Lumen des Leitungsmittels strömungsverbunden sind.

Es erweist sich weiter als vorteilhaft, wenn der Kopplungskörper so ausgebildet ist, dass nach dem Anspritzen wenigstens ein Lumen frei ausmündet, und dass durch Befestigen der Abschlusskappe das frei ausmündende Lumen nach außen dicht abgeschlossen ist.

Nach einer weiteren Ausführungsform der Erfindung ist der Kopplungskörper so ausgebildet, dass nach dem Anspritzen wenigstens zwei Lumen frei ausmünden und durch Befestigen der Abschlusskappe die frei ausmündenden Lumen nach außen dicht abgeschlossen und miteinander durchströmbar verbunden werden, indem die Abschlusskappe eine die beiden Lumen miteinander verbindende Ausnehmung aufweist. Dies bringt den Vorteil mit sich, dass die Lumen des Leitungsmittels bzw. des Kopplungskörpers an ihren distalen Enden und damit gewissermaßen totraumfrei miteinander verbunden werden.

Dies erweist sich als besonders vorteilhaft, wenn das eine Lumen als Spüllumen fungieren soll, da solchenfalls eine vollständige Spülung der Lumen sichergestellt werden kann, ohne dass sich Ablagerungen an nicht oder schwer durchströmbaren Totraumstellen bilden können. Die Ausbildung dieser Kommunikation an den distalen Enden der Lumen durch Befestigen und Eindichten der Abschlusskappe stellt eine sehr wirtschaftliche Möglichkeit der Herstellung des Kopplungskörpers bzw. der Anschlussvorrichtung dar. Es sei an dieser Stelle erwähnt, dass die Abschlusskappe selbst in einem Spritzgießverfahren als Spritzgussteil hergestellt werden kann.

Vorteilhafterweise sind das Leitungsmittel und der Kopplungskörper und vorzugsweise auch die Abschlusskappe aus einem biegsamen elastomeren Material, insbesondere aus Silikon oder auf Silikonbasis, ausgebildet, das eine Shore A - Härte von höchstens 65, insbesondere von höchstens 60, insbesondere von höchstens 50, insbesondere von höchstens 40, und weiter von wenigstens 10, insbesondere von wenigstens 15 aufweist.

Die Abschlusskappe ist vorteilhafterweise mit dem spritzgegossenen Material des Kopplungskörpers stoffschlüssig verbunden, insbesondere und vorzugsweise verklebt. Die Abschlusskappe schließt den Kopplungskörper vorzugsweise verrundet ab. Ein Übergang zur Abschlusskappe ist vorzugsweise im Wesentlichen fugen- und stufenfrei.

Zur Perfektionierung des Anfügens der Anschlusskappe wird in weiterer Ausbildung der Erfindung vorgeschlagen, dass der Kopplungskörper einen vorzugsweise dünnen Randbereich aufweist, in welchen die Abschlusskappe eingesetzt ist und der die Abschlusskappe in einer Umfangsrichtung kragenartig umgibt. Ein solcher Randbereich, der eine beispielhafte Wanddicke von 0,2 bis 1,5 mm aufweist, lässt sich im Spritzgießverfahren sehr einfach herstellen, zumal er nur einige wenige Millimeter, beispielsweise 1 bis 10 mm, insbesondere 1 bis 8 mm, insbesondere 1 bis 5 mm hervorzustehen braucht, um eine kragenartige Aufnahme für die Abschlusskappe zu bilden.

Es erweist sich weiter als vorteilhaft, wenn der Kopplungskörper mit einem ersten Bereich, welcher einen den Endabschnitt des Leitungsmittels zumindest teilweise umgebenden Teil, einen daran gegebenenfalls anschließenden Zwischenteil und die Abschlusskappe umfasst, und mit einem demgegenüber mit einer geringeren Dicke ausgebildeten und flächenhaft erstreckten zweiten Bereich ausgebildet ist. Der erwähnte Zwischenteil des Kopplungskörpers ergibt sich dann, wenn der Endabschnitt des Leitungsmittels sich nicht über im Wesentlichen die gesamte Längserstreckung des ersten Bereichs erstreckt, sondern nur einen verhältnismäßig kurzen Ansatz zum Anspritzen des Kopplungskörpers bildet. Solchenfalls werden die Lumen des Leitungsmittels im Inneren des Kopplungskörpers fortgesetzt durch im Zuge des Spritzgießens gebildete kanalbildende Ausnehmungen, mithin Lumen. Einer Ausbildung des Kopplungskörpers mit einem verhältnismäßig kurzen den Endabschnitt des Leitungsmittels umschließenden Teil und einem sich hieran anschließenden Zwischenteil, in dem die Lumen des Leitungsmittels fortgesetzt werden, wird der Vorzug gegeben. Solchenfalls erweist es sich als vorteilhaft, wenn der Endabschnitt des Leitungsmittels, also der mit Material des Kopplungskörpers umspritzte Bereich des Leitungsmittels, etwa 2 bis 20 mm, insbesondere 2 bis 15 mm und weiter insbesondere 3 bis 12 mm und weiter insbesondere 5 bis 10 mm in den Kopplungskörper hinein erstreckt ist.

Es erweist sich weiter als zweckmäßig, wenn der vorerwähnte flächenhaft erstreckte zweite Bereich des Kopplungskörpers mit einer Dicke von 0,1 bis 2 mm, insbesondere 0,1 bis 1,5 mm, insbesondere 0,1 - 1,0 mm, insbesondere von 0,1 - 0,8 mm, insbesondere von 0,1 - 0,5 mm ausgebildet ist.

Zweckmäßigerweise ist der zweite Bereich derart flächenhaft ausladend ausgebildet, dass eine Anlagefläche des zweiten Bereichs an die wundabgewandte Oberseite des Unterdruckverbands wenigstens 1,5 mal so groß, insbesondere wenigstens 1,8 mal so groß ist wie die Anlagefläche des ersten Bereichs. Die Anlagefläche wird dabei in der senkrechten Projektion auf die Erstreckungsebene des Kopplungskörpers betrachtet bzw. berechnet.

Nach einem weiteren Gedanken wird vorgeschlagen, dass der erste Bereich des Kopplungskörpers die Längserstreckung des Leitungsmittels fortsetzt und der zweite Bereich des Kopplungskörpers den ersten Bereich beidseits seiner Längserstreckung flankiert und in einer Draufsicht insbesondere schmetterlingsartig geformt ist, d.h. beidseits der Längserstreckung eine Einschnürung aufweist.

Weiter erweist es sich als vorteilhaft, wenn die maximale Dicke des ersten Bereichs des an das Leitungsmittel angespritzten Kopplungskörpers höchstens 3 mm, insbesondere höchstens 2 mm größer ist als die Dicke des Leitungsmittels und dass die Breite des ersten Bereichs des Kopplungskörpers höchstens 5 mm, insbesondere höchstens 3 mm, insbesondere höchstens 2 mm größer ist als die Breite des Leitungsmittels.

Weiter erweist es sich als besonders vorteilhaft, wenn die Dickenerstreckung des Leitungsmittels höchstens 7 mm, insbesondere höchstens 6 mm, insbesondere wenigstens 3 mm und weiter insbesondere 4-6 mm beträgt, wobei seine Breite quer zur Längserstreckung wenigstens 12 mm, insbesondere wenigstens 15 mm, insbesondere zwischen 15 mm und 30 mm, insbesondere zwischen 15 mm und 25 mm, insbesondere zwischen 15 mm und 22 mm, beträgt.

Es kann sich als vorteilhaft erweisen, wenn ein erstes Lumen des Leitungsmittels ein Spüllumen, d.h. ein Zuführlumen für fluide Spülmedien, insbesondere Luft, zu dem Kopplungskörper bildet und dass ein zweites Lumen des Leitungsmittels ein mit Unterdruck beaufschlagbares Sauglumen zum Abführen von fluiden Medien von der Wunde in Richtung auf eine Unterdruck erzeugende Einrichtung bildet, und wenn das zweite Lumen einen größeren Querschnitt aufweist als das erste Lumen. Das mit einem größeren Leitungsquerschnitt ausgebildete Sauglumen eignet sich solchenfalls zum Abführen von Wundsekreten mit darin oftmals enthaltenen partikelförmigen Bestandteilen.

Es erweist sich als besonders vorteilhaft, wenn der Kopplungskörper wenigstens drei nebeneinander erstreckte Lumen umfasst, wobei ein erstes Lumen als Spüllumen ausgebildet ist, wobei wenigstens ein zweites Lumen als Sauglumen ausgebildet ist und wobei ein drittes Lumen das Lumen zur Zuführung eines fluiden Mediums zur Wunde, d.h. in den Wundraum, bildet, und wenn das Spüllumen und das Sauglumen an ihren Enden strömungsmäßig miteinander verbunden sind. Weiter kann es sich als besonders vorteilhaft erweisen, wenn das als Spüllumen ausgebildete Lumen in Richtung auf die Wunde abgeschlossen, also ohne Öffnungen ausgebildet ist, und dann vorzugsweise erst über sein distales Ende mit den weiteren Lumen innerhalb des Kopplungskörpers strömungsmäßig verbunden ist. Dies kann - wie vorausgehend erläutert -vorteilhafterweise durch eine Strömungskommunikation über die Abschlusskappe realisiert werden.

Zum Anfügen der Anschlussvorrichtung an den Unterdruckverband erweist es sich als vorteilhaft, dass eine dem Unterdruckverband zugewandte Seite des Kopplungskörpers mit einer klebenden Schicht oder einer klebenden Beschichtung ausgebildet ist, um zum bestimmungsgemäßen Gebrauch eine klebende und gegen die Umgebung im wesentlichen unterdruckdichte Verbindung zu dem Unterdruckverband herzustellen.

Weiter erweist es sich als vorteilhaft, dass die klebende Schicht oder Beschichtung von einer wenigstens dreilagigen Haftvermittlungsschicht gebildet ist, die eine mittlere Traglage, eine an der Traglage gehaltene und dem Kopplungskörper zugewandte erste Kleberschicht und eine an der Traglage gehaltene und von dem Kopplungskörper abgewandte zweite Kleberschicht umfasst. Die Haftvermittlungsschicht ist so ausgebildet, dass sie die wenigstens eine Öffnung in dem Kopplungskörper nicht blockiert und dass die erste Kleberschicht und die zweite Kleberschicht von unterschiedlichen Klebematerialien mit unterschiedlichen Klebeeigenschaften gebildet sind. Hierbei erweist es sich als vorteilhaft, dass bei einer Ausbildung des Kopplungskörpers aus Silikon die erste Kleberschicht einen Silikonkleber umfasst. Weiter erweist es sich zumeist als vorteilhaft, wenn die zweite Kleberschicht einen Acrylatkleber umfasst, der dann in aller Regel geeignet ist, mit typischen Wundverbandmaterialien eine im Wesentlichen unterdruckdichte Verbindung einzugehen. Die erste und die zweite Kleberschicht haben vorzugsweise eine Dicke von 20 bis 400 µm. Bei der mittleren Traglage wird ein Vliesmaterial, ein Flachmaterial mit einer textilen Bindung, wie z. B. ein Gestrick, Gewirk oder Gewebe, oder eine Kunststofffolie, eine Metallfolie oder ein Verbundwerkstoff hieraus bevorzugt. Weiter erweist es sich als vorteilhaft, wenn die wundzugewandte Seite der zweiten Kleberschicht von einer ablösbaren Schutzschicht überfangen ist, die vorzugsweise zweiteilig und weiter vorzugsweise mit einer Anfasslasche und/oder einem über die zweite Kleberschicht überstehenden und ergreifbaren Bereich ausgestattet wird.

Weitere Einzelheiten, Merkmale und Vorteile der Erfindung ergeben sich aus den beigefügten Patentansprüchen und der zeichnerischen Darstellung und nachfolgenden Beschreibung bevorzugter Ausführungsformen der Erfindung. In der Zeichnung zeigt:
Figur 1 eine Draufsicht auf eine erfindungsgemäße Anschlussvorrichtung zur Verwendung bei der Unterdruckbehandlung von Wunden, sowie eine angedeutete Wunde mit einem Unterdruckverband;
Figur 2 eine Schnittansicht der Anschlussvorrichtung nach Figur 1 mit Schnittebene II-II;
Figur 3 eine Schnittansicht der Anschlussvorrichtung nach Figur 1 mit Schnittebene III-III;
Figur 4 eine Schnittansicht einer weiteren Ausführungsform der Anschlussvorrichtung mit entsprechender Schnittebene III-III;
Figur 5 eine Draufsicht auf eine weitere Ausführungsform einer erfindungsgemäßen Anschlussvorrichtung mit angedeuteter Wunde und Unterdruckverband;
Figur 6 eine Schnittansicht der Anschlussvorrichtung nach Figur 5 mit Schnittebene II-II
Figur 7 eine Schnittansicht der Anschlussvorrichtung nach Figur 5 mit Schnittebene III-III.

Die Figuren zeigen eine insgesamt mit dem Bezugszeichen 2 bezeichnete erfindungsgemäße Anschlussvorrichtung zur Verwendung bei der Unterdrucktherapie von Wunden. Hierfür ist die Anschlussvorrichtung 2 mit einer nicht dargestellten unterdruckerzeugenden Einrichtung verbindbar, die typischerweise mit einem Flüssigkeitsaufnahmebehälter zum Abscheiden und Sammeln von aus dem Wundraum abgesaugten Wundsekreten und Spülmedien zusammenwirkt. Die Anschlussvorrichtung dient zum unterdruckdichten Ankoppeln an eine die Wunde 10 wiederum im Wesentlichen unterdruckdicht gegen die Umgebung abschließenden Unterdruckverband 12, wobei eine Unterdruckkommunikation zum Wundraum hergestellt wird.

Die erfindungsgemäße Anschlussvorrichtung 2 umfasst ein insgesamt mit dem Bezugszeichen 4 bezeichnetes Leitungsmittel und einen Kopplungskörper 6. Der Kopplungskörper 6 ist auf noch näher zu beschreibende erfindungsgemäße Weise an einen wundseitigen Endabschnitt 8 des Leitungsmittels 4 in einem Kunststoff-Spritzgießverfahren angespritzt.

Ein wundabgewandtes Ende des Leitungsmittels 4 ist mit der nicht dargestellten unterdruckerzeugenden Einrichtung verbindbar.

Der Kopplungskörper 6 umfasst einen im beispielhaft dargestellten Fall blockförmig anmutenden ersten Bereich 16, der die Längserstreckung des Leitungsmittels 4 fortsetzt, und einen demgegenüber mit einer wesentlich geringeren Dicke ausgebildeten und flächenhaft erstreckten zweiten Bereich 18, welcher sich ausgehend von dem ersten Bereich 16 vorzugsweise beidseitig und weiter vorzugsweise auf wenigstens drei Seiten des ersten Bereichs 16 flächenhaft wegerstreckt, damit eine große Anlagefläche des Kopplungskörpers 6 an den Unterdruckverband 12 realisiert werden kann.

Der erste Bereich 16 des Kopplungskörpers 6 umfasst einen den Endabschnitt 8 des Leitungsmittels 4 zumindest teilweise umgebenden Teil 20, einen daran in einer Längsrichtung 22 anschließenden Zwischenteil 24 und eine Abschlusskappe 26. Der den Endabschnitt umgebende Teil 20, der Zwischenteil 24 und der flächenhaft erstreckte zweite Bereich 18 des Kopplungskörpers 6 sind einstückig aus einem biegsamen elastomeren Material, vorzugsweise aus Silikon oder auf Silikonbasis, hergestellt. Hierfür wird das Leitungsmittel 4 bzw. der wundseitige Endabschnitt 8 des Leitungsmittels 4 in eine nicht dargestellte Spritzgießform eingebracht, und das den Kopplungskörper 6 bildende elastomere Material wird an den Endabschnitt 8 des Leitungsmittels 4 angespritzt. Hierbei ist die Spritzgießform derart ausgebildet bzw. ausgestattet, dass Strömungskanäle bildende Lumen 28, 30, 31 des Leitungsmittels 4 eine Fortsetzung innerhalb des Kopplungskörpers 6 finden. Es müssen im Spritzgießwerkzeug also entsprechende hohlraum- oder kanalbildende Mittel, insbesondere in Form von Stangen oder dergleichen, vorgesehen werden. Obschon nicht dargestellt, wäre es auch denkbar, dass sich das Leitungsmittel 4 bzw. dessen Endabschnitt 8 bis zu der Abschlusskappe 26 erstreckt. Solchenfalls wäre der den Endabschnitt 8 des Leitungsmittels 4 zumindest teilweise umgebende Teil 20 in Längsrichtung 22 länger ausgebildet und der Zwischenteil 24 wäre entbehrlich oder sehr viel kürzer ausgebildet. Es hat sich indessen als vorteilhaft erwiesen, wenn das Leitungsmittel 4 nur mit einem verhältnismäßig kurzen Endabschnitt 8 in den Kopplungskörper 6 einragt, da solchenfalls die Gestaltungsfreiheit des Kopplungskörpers 6 innerhalb des Zwischenteils 24 größer ist und bereits beim Spritzgießvorgang Öffnungen 32 in einer dem Unterdruckverband zugewandten Wandung 48 des Kopplungskörpers 6 ausgebildet werden können.

Der Zwischenteil 24 wird also in seinem Inneren mit in
quer zur Längsrichtung 22 voneinander abgetrennten Lumen 36, 38, 40 ausgebildet, die sich wie aus den Figuren ersichtlich an die Lumen 28, 30, 31 des Leitungsmittels 4 anschließen.

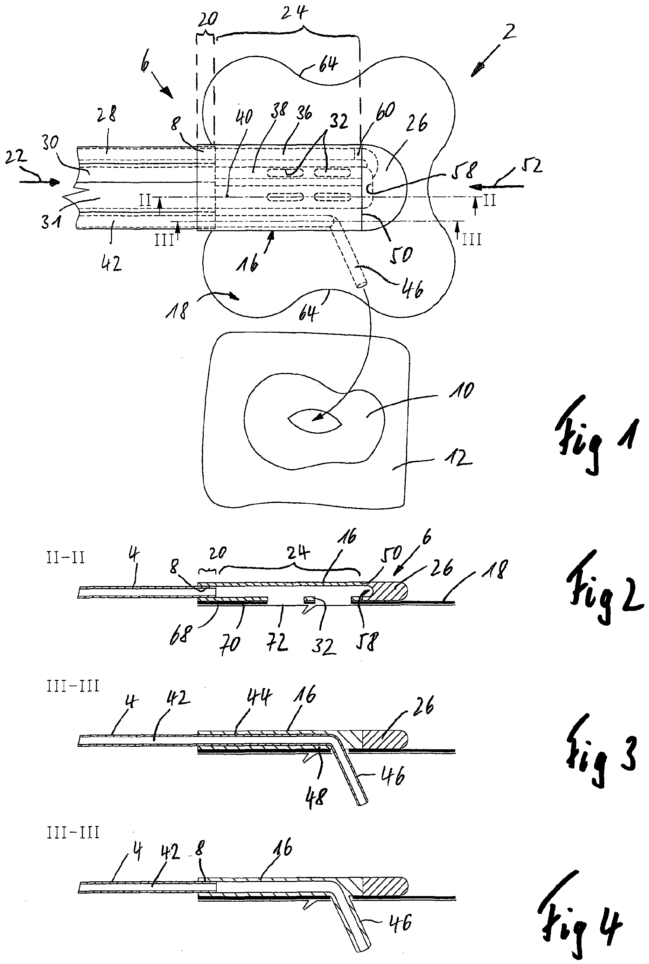

Man erkennt aus Figur 1 ferner, dass in dem Leitungsmittel 4 ein weiteres Lumen 42 ausgebildet ist, das zum Zuführen eines fluiden und insbesondere Wirkstoffe enthaltenden Mediums zum Wundraum dient. Wie aus Figur 3 ersichtlich ist, setzt sich das Leitungsmittel 4 mit einem das Lumen 42 bildenden und begrenzenden Leitungsabschnitt 44 durch den Kopplungskörper 6 hindurch fort. Dieser Leitungsabschnitt 44 wird also ebenfalls mit elastomerem Material umspritzt. Im beispielhaft dargestellten Fall ist dieser Leitungsabschnitt 44 in Richtung auf den Wundraum abgebogen und bildet einen Fluideinleitungsabschnitt 46, der über eine dem Unterdruckverband 10 zugewandte Wandung 48 des Kopplungskörpers 6 vorsteht.

In Figur 4 ist eine weitere Ausführungsform dargestellt, bei der das Leitungsmittel 4 über seine gesamte Breite wie in Figur 2 dargestellt, endet, so dass der vorerwähnte Leitungsabschnitt 44 nicht vorgesehen ist, sondern das Lumen 42 zum Zuführen eines fluiden Mediums durch einen entsprechend ausgebildeten lumenbildenden Hohlraum innerhalb des Kopplungskörpers 6 fortgesetzt und begrenzt wird. Solchenfalls kann der Fluideinleitungsabschnitt 46 entweder wie in Figur 4 dargestellt einstückig mit dem elastomeren Material des Kopplungskörpers 6 im Spritzgießverfahren hergestellt werden, oder er wird von einem zusätzlichen Leitungsstück gebildet, welches in eine entsprechend ausgebildete Öffnung in der Wandung 48 des Kopplungskörpers eingesetzt wird.

Man erkennt insbesondere aus Figuren 1 und 2, dass die Lumen 36, 38, 40 des Kopplungskörpers 6 an einer der Abschlusskappe 26 zugewandten Stirnseite 50 des Zwischenteils 24 frei ausmünden. Sie werden durch Anfügen und unterdruckdichtes Befestigen der Abschlusskappe 26 zur Umgebung hin unterdruckdicht abgeschlossen. Die Abschlusskappe 26, die klemmschlüssig oder vorzugsweise stoffschlüssig unlösbar, insbesondere mittels Kleber, befestigt wird, komplettiert den Kopplungskörper 6 der erfindungsgemäßen Anschlussvorrichtung 2. Beim Anspritzen des den Kopplungskörper 6 bildenden elastomeren Materials kann ein nicht dargestellter entgegen einer Aufsteckrichtung 52 der Abschlusskappe 26 erstreckter Randbereich gebildet werden, der eine Aufnahmeöffnung für die Abschlusskappe 26 begrenzt. Dieser Randbereich umgibt dann die Abschlusskappe 26 kragenförmig. Er ist vorzugsweise verhältnismäßig dünnwandig ausgebildet, beispielhaft nur 0,5 bis 1,5 mm dick. Der Randbereich erstreckt sich vorzugsweise über drei Seiten, so dass die Aufnahmeöffnung auf der vierten Seite von dem flächenhaft erstreckten zweiten Bereich 18 des Kopplungskörpers 6 begrenzt wird.

Die Abschlusskappe 26 ist so ausgebildet, dass sie im Wesentlichen fugenfrei und stufenfrei an den Zwischenteil 24 des Kopplungskörpers 6 anschließt.

Die Abschlusskappe 26 ist weiterhin mit einer insgesamt mit dem Bezugszeichen 58 bezeichneten Ausnehmung ausgebildet, mittels derer eine Strömungskommunikation zwischen wenigstens zwei, im beispielhaft dargestellten Fall zwischen allen drei Lumen 36, 38, 40 erreicht wird, und zwar ausgehend von deren distal mündenden Öffnungen im Bereich der Stirnseite 50. Die Ausnehmung 58 könnte in mehrfacher Weise, im einfachsten Fall beispielsweise in Form einer langlochförmigen Vertiefung realisiert sein, die sich dann an die Stirnseite des Zwischenteils 24 anschließt. Die Abschlusskappe 26 ist mit einem tubusförmigen Ansatz 60 ausgebildet, der beim Aufstecken der Abschlusskappe 26 in das komplementär hierzu ausgebildete Lumen 36 in dem Zwischenteil 24 dichtend eingreift. Dieser tubusförmige Ansatz 60 mündet im Inneren der Abschlusskappe 26 in die quer verlaufende Ausnehmung 58, die sich zur Stirnseite 50 des Zwischenteils 24 langlochförmig öffnet. Auf diese Weise besteht eine Strömungskommunikation zwischen den Lumen 36, 38, 40. Im beispielhaft dargestellten Fall dient das Lumen 28 des Leitungsmittels 4 und das daran anschließende Lumen 36 innerhalb des Kopplungskörpers 6 als Spüllumen zur Zuführung eines fluiden Spülmediums (Luft oder Spülflüssigkeit). Fluide Spülmedien werden auf diese Weise an die distalen Mündungsöffnungen der Lumen 38, 40 in der Stirnseite 50 des Zwischenteils 24 herangeführt. Hierdurch wird eine totraumfreie Spülung möglich; es gibt also keinen Leitungsbereich, der nicht unmittelbar entlang des Strömungswegs von der Strömung erfasst würde, was im Hinblick auf eine bestimmungsgemäße Funktion der Anschlussvorrichtung der mit ihr durchgeführten Unterdruckwundtherapie und im Hinblick auf eine Reduzierung von bakteriellem Wachstum und von Infektionen als besonders wertvoll angesehen wird. Der Abschlusskappe 26 kommt also eine Doppelfunktion zu, nämlich zum einen komplettiert sie den Kopplungskörper 6 und verschließt die nach dem Spritzgießverfahren ausmündenden Öffnungen der Lumen 36, 38, 40, und zum anderen wird durch die Abschlusskappe 26 eine Strömungskommunikation zwischen den Lumen 36, 38, 40 realisiert.

Zur Unterdruckkommunikation mit dem Wundraum sind die schon erwähnten Öffnungen 32 in der dem Unterdruckwundverband zugewandten Wandung 34 des Kopplungskörpers 6 ausgebildet. Man erkennt aus den Figuren, dass das als Spüllumen fungierende Lumen 36 des Kopplungskörpers nicht über eine solche Öffnung verfügt, was bevorzugt, jedoch nicht zwingend ist. Die im Bereich der Längserstreckung der Lumen 38, 40 vorgesehenen Öffnungen 32 sind in bevorzugter Weise langlochförmig ausgebildet und haben eine beispielhaft bevorzugte Länge von 8 bis 12 mm. Im beispielhaft dargestellten Fall sind entlang der Erstreckung jedes Lumens 38, 40 zwei solcher langlochförmiger Öffnungen ausgebildet. Im Betrieb kommunizieren die Lumen 38, 40 des Kopplungskörpers über diese Öffnungen 32 und über wenigstens eine Öffnung in dem Unterdruckverband hindurch mit dem Wundraum. Typischerweise werden die Lumen 38, 40 des Kopplungskörpers 6 über die Lumen 30,31 in dem Leitungsmittel 4 mit Unterdruck beaufschlagt; die Lumen 38, 40 fungieren also typischerweise als Sauglumen zum Anlegen von Unterdruck und zum Abführen von Wundsekreten, Spülflüssigkeit oder sonstigen zugeführten fluiden Medien.

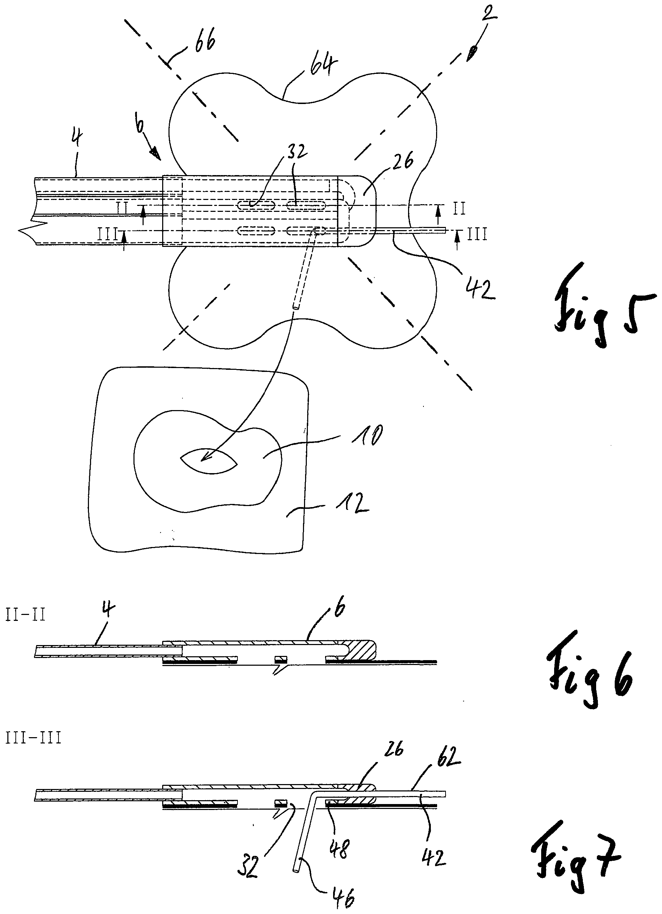

Die Figuren 5 bis 7 zeigen eine weitere Ausführungsformen der erfindungsgemäßen Anschlussvorrichtung, bei der das Lumen 42 zum Zuführen eines fluiden Mediums zum Wundraum nicht von dem Leitungsmittel 4 gebildet ist, sondern von einer zusätzlichen Fluidzuführleitung 62, die jedoch auch ungefähr parallel zur flächenhaften Erstreckung der Anschlussvorrichtung 2 an den Kopplungskörper 6 herangeführt ist. Die Fluidzuführleitung 62 könnte beispielsweise neben dem Leitungsmittel 4 und parallel zu diesem an den Kopplungskörper 6 herangeführt sein (nicht dargestellt). Im beispielhaft dargestellten Fall erstreckt sich die Fluidzuführleitung 62 durch die Abschlusskappe 26 hindurch in das Innere des Kopplungskörpers 6 und von dort durch eine Öffnung 32 in der dem Wundverband zugewandten Wandung 48 des Kopplungskörpers 6 in den Wundraum. Das Ende der Fluidzuführleitung 62 bildet also einen Fluideinleitungsabschnitt 46, der von der wundzugewandten Seite des Kopplungskörpers 6 vorsteht und durch eine weitere Öffnung in den Wundverband 12 hindurch in den Wundraum der Wunde 10 einragen kann. Es ist denkbar, dass die Fluidzuführleitung 62 lediglich in eine Durchgangsöffnung der Abschlusskappe 6 eingesteckt ist und dass der Fluideinleitungsabschnitt 46 dann entweder einstückig mit dem Kopplungskörper 6 ausgebildet ist oder von einem in eine Öffnung des Kopplungskörpers 6 eingesteckten Leitungsabschnitt gebildet ist.

Bei der Ausführungsform nach Figuren 5-7 ist das Leitungsmittel 4 vorzugsweise nur dreilumig ausgebildet.

Es wird nun die in den Figuren beispielhaft dargestellte Gestalt des flächenhaft erstreckten zweiten Bereichs 18 des Kopplungskörpers 6 beschrieben. Diese Gestalt oder Form lässt sich in der Draufsicht am ehesten als schmetterlingsförmig bezeichnen, da dieser zweite Bereich 18 in Längsrichtung 22 betrachtet beidseits eine Einschnürung 64 aufweist. Auf diese Weise sind die Abmessungen des Kopplungskörpers 6 bzw. seines zweiten Bereichs 18 entlang von angedeuteten Diagonalen 66, die einen Winkel von etwa 45° zur Längsrichtung 22 bilden, größer als in der Längsrichtung 22 und senkrecht hierzu. Im beispielhaft und bevorzugt dargestellten Fall erstreckt sich der flächenhaft erstreckte dünne zweite Bereich 18 nach drei Seiten über den ebenfalls eher flach bauenden, jedoch als blockförmig zu bezeichnenden ersten Bereich 16 des Kopplungskörpers 6 hinaus. Auf diese Weise ist eine sehr gute Anbindung an einen nicht dargestellten Unterdruckwundverband erreichbar, da die auftretenden Kräfte über eine große Fläche gleichmäßig verteilt und daher ohne lokale Kraftspitzen in diejenigen die Wunde umgebenden Bereiche des Wundverbands und der Körperoberfläche des Patienten eingeleitet werden.

Zur Anbindung an den Unterdruckverband umfasst der Kopplungskörper auf seiner dem Unterdruckverband zugewandten Seite 68 eine klebende Schicht 70 und eine ablösbare Decklage 72. Die klebende Schicht 70 ist bevorzugtermaßen als eine wenigstens dreilagige Haftvermittlungsschicht ausgebildet, die eine mittlere Traglage, eine an der Traglage gehaltene und dem Kopplungskörper zugewandte erste Kleberschicht und eine an der Traglage gehaltene und von dem Kopplungskörper abgewandte zweite Kleberschicht umfasst. Die Kleberschichten sind jeweils im Hinblick auf die Ausbildung einer Haftverbindung mit dem Material des Kopplungskörpers bzw. mit dem Material des Unterdruckverbands optimiert ausgebildet. Hinsichtlich weiterer Merkmale dieser wenigstens dreilagigen Haftvermittlungsschicht wird Bezug genommen auf die nicht vorveröffentlichte DE 10 2011 108 726.9.

## Patentansprüche

1. Anschlussvorrichtung (2) zur Verwendung bei der Unterdrucktherapie von Wunden, mit einem mit Unterdruck und/oder fluiden Medien beaufschlagbaren biegsamen Leitungsmittel (4) mit wenigstens zwei Lumen (28, 30, 31) und mit einem mit dem Leitungsmittel (4) verbundenen Kopplungskörper (6), wobei der Kopplungskörper auf einen die Wunde (10) überfangenden und gegen die Atmosphäre dichtend abschließenden Unterdruckverband (12) aufbringbar ist, wobei das Leitungsmittel (4) durch wenigstens eine Öffnung (32) hindurch mit dem Wundraum kommuniziert, **dadurch gekennzeichnet, dass** der Kopplungskörper (6) aus einem elastomeren biegsamen Material ausgebildet ist, welches unlösbar an einen wundseitigen Endabschnitt (8) des Leitungsmittels (4) angespritzt ist, dass an dem Kopplungskörper (6) ein Fluideinleitungsabschnitt (46) ausgebildet ist, der über die dem Unterdruckverband (12) zugewandte Wandung (48) des Kopplungskörpers (6) vorsteht und durch die Öffnung in dem Unterdruckverband (12) hindurch in den Wundraum oder in eine dort vorgesehene Wundeinlage einragen kann, wobei dieser Fluideinleitungsabschnitt (46) über das Innere des Kopplungskörpers (6) mit einem weiteren Lumen (42) zum Zuführen eines fluiden Mediums strömungsverbunden ist, welches Lumen (42) entweder innerhalb des Leitungsmittels (4) ausgebildet ist oder von einer zusätzlichen an den Kopplungskörper (6) herangeführten Fluidzuführleitung (62) gebildet ist.

2. Anschlussvorrichtung nach Anspruch 1, **dadurch gekennzeichnet, dass** der Fluideinleitungsabschnitt (46) von der an den Kopplungskörper (6) herangeführten Fluidzuführleitung (62) gebildet ist, die sich durch den Kopplungskörper (6) hindurcherstreckt, wobei das den Kopplungskörper (6) bildende elastomere biegsame Material auch an die Fluidzuführleitung angespritzt sein kann.

3. Anschlussvorrichtung nach Anspruch 1, **dadurch gekennzeichnet, dass** der Fluideinleitungsabschnitt (46) einstückig mit dem Kopplungskörper (6) im Spritzgießverfahren ausgebildet ist.

4. Anschlussvorrichtung nach Anspruch 1, **dadurch gekennzeichnet, dass** der Fluideinleitungsabschnitt (46) als von dem Kopplungskörper (6) separat hergestelltes Leitungsbauteil ausgebildet ist, das in eine mit dem Inneren des Kopplungskörpers strömungsverbundene Öffnung in der dem Unterdruckverband zugewandten Wandung des Kopplungskörpers vorzugsweise dichtend eingesetzt und darin fixiert ist.

5. Anschlussvorrichtung nach einem oder mehreren der vorstehenden Ansprüche, **dadurch gekennzeichnet, dass** zur weiteren Ausbildung des Kopplungskörpers (6) an das angespritzte Material eine Abschlusskappe (26) angefügt und vorzugsweise unlösbar befestigt ist, wodurch der Kopplungskörper (6) nach außen dicht abgeschlossen ist.

6. Anschlussvorrichtung nach Anspruch 5, **dadurch gekennzeichnet, dass** die zusätzliche an den Kopplungskörper (6) herangeführte Fluidzuführleitung (62) durch die Abschlusskappe (26) hindurch mit dem Inneren des Kopplungskörpers (6) strömungsverbunden ist.

7. Anschlussvorrichtung nach Anspruch 5, **dadurch gekennzeichnet, dass** der Kopplungskörper (6) so ausgebildet ist, dass nach dem Anspritzen wenigstens zwei Lumen (36, 38, 40) frei ausmünden, und dass durch Befestigen der Abschlusskappe (26) die frei ausmündenden Lumen (36, 38, 40) nach außen dicht abgeschlossen und miteinander durchströmbar verbunden werden, indem die Abschlusskappe (26) eine die beiden Lumen miteinander verbindende Ausnehmung (58) aufweist.

8. Anschlussvorrichtung nach Anspruch 5, **dadurch gekennzeichnet, dass** der Kopplungskörper (6) einen vorzugsweise dünnen Randbereich (54) aufweist, in welchen die Abschlusskappe (26) eingesetzt ist und der die Abschlusskappe (26) in einer Umfangsrichtung kragenartig umgibt.

9. Anschlussvorrichtung nach Anspruch 5, **dadurch gekennzeichnet, dass** der Kopplungskörper (6) mit einem ersten Bereich (16), welcher einen den Endabschnitt (8) des Leitungsmittels (4) zumindest teilweise umgebenden Teil (20), einen daran gegebenenfalls anschließenden Zwischenteil (24) und die Abschlusskappe (26) umfasst, und mit einem demgegenüber mit einer geringeren Dicke ausgebildeten und flächenhaft erstreckten zweiten Bereich (18) ausgebildet ist.

10. Anschlussvorrichtung nach Anspruch 9, **dadurch gekennzeichnet, dass** der zweite Bereich (18) des Kopplungskörpers (6) mit einer Dicke von 0,1 - 2,0 mm, insbesondere 0,1 - 1,5 mm, insbesondere 0,1 - 1,0 mm, insbesondere von 0,1 - 0,8 mm, insbesondere von 0,1 - 0,5 mm ausgebildet ist.

11. Anschlussvorrichtung nach Anspruch 9 oder 10, **dadurch gekennzeichnet, dass** der zweite Bereich (18) derart flächenhaft ausladend ausgebildet ist, dass eine Anlagefläche des zweiten Bereichs (18) an die wundabgewandte Oberseite des Unterdruckverbands wenigstens 1,5 mal so groß, insbesondere wenigstens 1,8 mal so groß ist wie die Anlagefläche des ersten Bereichs (16).

12. Anschlussvorrichtung nach einem oder mehreren der Ansprüche 9-11, **dadurch gekennzeichnet, dass** die maximale Dicke des ersten Bereichs (16) des an das Leitungsmittel (8) angespritzten Kopplungskörpers (6) höchstens 3 mm, insbesondere höchstens 2 mm größer ist als die Dicke des Leitungsmittels (4) und dass die Breite des ersten Bereichs (16) des Kopplungskörpers (6) höchstens 5 mm, insbesondere höchstens 3 mm, insbesondere höchstens 2 mm größer ist als die Breite des Leitungsmittels (4).

13. Anschlussvorrichtung nach einem oder mehreren der vorstehenden Ansprüche, **dadurch gekennzeichnet, dass** die Dickenerstreckung des Leitungsmittels (4) höchstens 7 mm, insbesondere höchstens 6 mm, insbesondere wenigstens 3 mm und weiter insbesondere 4-6 mm beträgt, wobei die Breite quer zur Längserstreckung wenigstens 12 mm, insbesondere wenigstens 15 mm, insbesondere zwischen 15 mm und 30 mm, insbesondere zwischen 15 mm und 25 mm, insbesondere zwischen 15 mm und 22 mm, beträgt.

14. Anschlussvorrichtung nach einem oder mehreren der vorstehenden Ansprüche, **dadurch gekennzeichnet, dass** der Kopplungskörper (6) wenigstens drei nebeneinander erstreckte Lumen (36, 38, 40) umfasst, wobei ein Lumen (36) als Spüllumen ausgebildet ist, wobei wenigstens ein Lumen als Sauglumen ausgebildet ist und wobei ein Lumen das Lumen zur Zuführung eines fluiden Mediums zur Wunde bildet, und dass das Spüllumen und das Sauglumen an ihren Enden strömungsmäßig miteinander verbunden sind.

15. Anschlussvorrichtung nach einem oder mehreren der vorstehenden Ansprüche, **dadurch gekennzeichnet, dass** eine dem Unterdruckverband zugewandte Seite des Kopplungskörpers (6) mit einer klebenden Schicht oder einer klebenden Beschichtung ausgebildet ist, um zum bestimmungsgemäßen Gebrauch eine klebende und gegen die Umgebung im wesentlichen unterdruckdichte Anbindung an den Unterdruckverband herzustellen.

## Claims

1. Connection device (2) for use for the negative pressure therapy of wounds, with a flexible conducting means (4) that can be impinged with negative pressure and/or fluid media with at least two lumens (28, 30, 31) and with a coupling body (6) which is connected to the conducting means (4), wherein the coupling body can be attached to a negative pressure bandage which covers the wound (10) and seals the wound against the atmosphere (12), wherein the conducting means (4) communicates with the wound space through at least one opening (32), **characterized in that** the coupling body (6) is made of a flexible material, which is non-detachably molded onto a wound side end section (8) of the conducting means (4), **in that** a fluid inlet section (46) is formed on the coupling body (6) which fluid inlet section (46) protrudes over the walling (48) of the coupling body (6) that faces the negative pressure bandage (12) and can protrude through the opening in the negative pressure bandage (12) into the wound space or into a wound insert provided there, wherein this fluid inlet section (46) is fluidly connected with a further lumen (42) via the interior of the coupling body (6) for delivering a fluid medium, which lumen (42) is either formed inside the conducting means (4) or is formed by an additional fluid delivery line (62) that leads to the coupling body (6).

2. Connection device according to claim 1, **characterized in that** the fluid inlet line (46) is formed by the fluid delivery line (62) which leads to the coupling body (6), which fluid delivery line (62) extends through the coupling body (6), wherein the elastomeric material which forms the coupling body (6) can also be molded onto the fluid delivery line.

3. Connection device according to claim 1, **characterized in that** the fluid inlet section (46) is formed one-piece with the coupling body (6) in the injection molding process.

4. Connection device according to claim 1, **characterized in that** the fluid inlet section (46) is formed as conducting component that is produced separate from the coupling body (6), and is inserted preferably sealingly into an opening in the walling of the coupling body which walling faces the negative pressure bandage, which opening is fluidly connected with the interior of the coupling body.

5. Connection device according to one or more of the preceding claims, **characterized in that** for further formation of the coupling body (6) an end cap (26) is attached to the molded on material and preferably non detachably fastened, whereby the coupling body (6) is sealed tight outwardly.

6. Connection device according to claim 5, **characterized in that** the additional fluid delivery line (62) that leads to the coupling body (6) is fluidly connected with the interior of the coupling body (6) through the end cap (26).

7. Connection device according to claim 5, **characterized in that** the coupling body (6) is configured so that after the melding on, at least two lumens (36, 38, 40) end freely, and **in that** by fastening of the end cap (26) the freely ending lumens (36, 38, 40) are sealed tight to the outside and to each other circulatingly, **in that** the end cap (26) has a recess which interconnects the two lumens.

8. Connection device according to claim 5, **characterized in that** the coupling body (6) has a preferably thin border region, in which the end cap (26) is inserted and which surrounds the end cap (26) collar like in a circumferential direction.

9. Connection device according to claim 5, **characterized in that** the coupling body (6) is configured with a first region (16) which includes a part (20) which at least partially surrounds the end section (8) of the conducting means (4), an intermediate part (24) adjoining thereon and the end cap, and with a second region (18) which relative to this is configured with a smaller thickness and extends two dimensionally.

10. Connection device according to claim 9, **characterized in that** the second region (18) of the coupling body (6) is formed with a thickness of 0.1 - 2.0 mm, in particular of 0.1 - 1.5 mm, in particular of 0.1 - 1.0 mm, in particular of 0.1 - 0.8 nm, in particular of 0.1 - 0.5 mm.

11. Connection device according to claim 9 or 10, **characterized in that** the second region (18) extends two dimensionally so that a contact surface of the second region (18) to the wound averted top side of the negative pressure bandage is at least 1.5 times, in particular at least 1.8 times as large as the contact surface of the first region (16).

12. Connection device according to one or more of the preceding claims 9 - 11, **characterized in that** the maximal thickness of the first region (16) of the coupling body (6) which is molded onto the conducting means (8) is at most 3 mm, in particular at most 2 mm greater than the thickness of the conducting means (4) and **in that** the width of the first region (16) of the coupling body (6) is at most 5 mm, in particular at most 3 mm, in particular at most 2 mm greater than the width of the conducting means (4).

13. Connection device according to one or more of the preceding claims, **characterized in that** the thickness extent of the conducting means (4) is at most 7 mm, in particular at most 6 mm, in particular at least 3 mm and further in particular 4 - 6 mm, wherein the width transverse to the longitudinal extent is at least 12 mm, in particular at least 15 mm, in particular between 15 mm and 30 mm, in particular between 15 mm and 25 mm, in particular between 15 mm and 22 mm.

14. Connection device according to one or more of the preceding claims, **characterized in that** the coupling body (6) includes at least three lumens (36, 38, 40) that extend adjacent one another, wherein one lumen (36) is configured as rinsing lumen, wherein at least one lumen is configured as suction lumen and wherein one lumen forms the lumen for delivering a fluid medium to the wound, and **in that** the rinsing lumen and the suction lumen are fluidly connected at their ends.

15. Connection device according to one or more of the preceding claims, **characterized in that** a side of the coupling body (6) which faces the negative pressure bandage is configured with an adhesive layer or an adhesive coating, in order to generate an adhesive connection to the negative pressure bandage for the intended use, which adhesive connection is essentially negative pressure tight.

## Revendications

1. Dispositif de raccordement (2) destiné à être utilisé dans le traitement de plaies par pression négative, comprenant un moyen de conduite (4) souple qui peut être alimenté en pression négative et/ou en milieux fluides et qui comprend au moins deux lumières (28, 30, 31), ainsi qu'un corps d'accouplement (6) relié au moyen de conduite (4), dans lequel le corps d'accouplement (6) peut être appliqué sur un pansement à pression négative (12) qui recouvre la plaie (10) et isole celle-ci de façon étanche par rapport à l'atmosphère, dans lequel ledit moyen de conduite (4) communique par au moins un orifice (32) avec la zone de la plaie, **caractérisé par le fait que** le corps d'accouplement (6) est réalisé en un matériau élastomère souple qui est surmoulé par injection de manière inamovible sur une portion d'extrémité côté plaie (8) du moyen de conduite (4), que sur le corps d'accouplement (6) est réalisée une portion d'introduction de fluide (46) qui dépasse la paroi (48) du corps d'accouplement (6) laquelle montre vers le pansement à pression négative (12) et qui peut se projeter, à travers l'orifice ménagé dans le pansement à pression négative (12), dans la zone de la plaie ou dans un insert pour plaie y prévu, dans lequel cette portion d'introduction de fluide (46) est reliée fluidiquement via l'intérieur du corps d'accouplement (6) à une autre lumière (42) pour amener un milieu fluide, laquelle lumière (42) soit est réalisée à l'intérieur du moyen de conduite (4) soit est formée par une conduite d'alimentation en fluide (62) supplémentaire amenée au corps d'accouplement (6).

2. Dispositif de raccordement selon la revendication 1, **caractérisé par le fait que** ladite portion d'introduction de fluide (46) est formée par la conduite d'alimentation en fluide (62) amenée au corps d'accouplement (6), qui s'étend à travers le corps d'accouplement (6), dans lequel le matériau élastomère souple formant le corps d'accouplement (6) peut être surmoulé par injection aussi sur la conduite d'alimentation en fluide.

3. Dispositif de raccordement selon la revendication 1, **caractérisé par le fait que** la portion d'introduction de fluide (46) est réalisée d'un seul tenant avec le corps d'accouplement (6) dans le procédé de moulage par injection.

4. Dispositif de raccordement selon la revendication 1, **caractérisé par le fait que** la portion d'introduction de fluide (46) est réalisée en tant que composant de conduite qui est fabriqué de manière séparée du corps d'accouplement (6) et qui est inséré de préférence à étanchéité dans un orifice et y fixé, orifice qui se trouve dans la paroi du corps d'accouplement laquelle montre vers le pansement à pression négative et qui est relié fluidiquement à l'intérieur du corps d'accouplement.

5. Dispositif de raccordement selon l'une ou plusieurs des revendications précédentes, **caractérisé par le fait que**, pour l'autre formation du corps d'accouplement (6), un capuchon de fermeture (26) est joint et, de préférence, fixé de manière inamovible sur le matériau surmoulé par injection, ce par quoi le corps d'accouplement (6) est fermé de manière étanche vers l'extérieur.

6. Dispositif de raccordement selon la revendication 5, **caractérisé par le fait que** ladite conduite d'alimentation en fluide (46) supplémentaire amenée au corps d'accouplement (6) est reliée fluidiquement, à travers le capuchon de fermeture (26), à l'intérieur du corps d'accouplement (6).

7. Dispositif de raccordement selon la revendication 5, **caractérisé par le fait que** le corps d'accouplement (6) est réalisé de telle sorte que, après le surmoulage par injection, au moins deux lumières (36, 38, 40) débouchent librement, et que, en fixant ledit capuchon de fermeture (26), les lumières (36, 38, 40) débouchant librement sont fermées de manière étanche vers l'extérieur et sont reliées entre elles de manière à pouvoir être traversées par **le fait que** le capuchon de fermeture (26) présente un évidement (58) reliant entre elles les deux lumières.

8. Dispositif de raccordement selon la revendication 5, **caractérisé par le fait que** le corps d'accouplement (6) présente une zone marginale (48) de préférence mince dans laquelle le capuchon de fermeture (26) est inséré et qui entoure à la manière d'une collerette ledit capuchon de fermeture (26) dans une direction circonférentielle.

9. Dispositif de raccordement selon la revendication 5, **caractérisé par le fait que** le corps d'accouplement (6) est réalisé avec une première zone (16) qui comprend une partie (20) entourant au moins en partie la portion d'extrémité (8) du moyen de conduite (4), une partie intermédiaire (24) le cas échéant adjacente à cette première ainsi que le capuchon de fermeture (26), et avec une deuxième zone (18) présentant une épaisseur plus faible par rapport à la première et étendue en nappe.

10. Dispositif de raccordement selon la revendication 9, **caractérisé par le fait que** la deuxième zone (18) du corps d'accouplement (6) est configurée avec une épaisseur comprise entre 0,1 et 2,0 mm, en particulier entre 0,1 et 1,5 mm, en particulier entre 0,1 et 1,0 mm, en particulier entre 0,1 et 0,8 mm, en particulier entre 0,1 et 0,5 mm.

11. Dispositif de raccordement selon la revendication 9 ou 10, **caractérisé par le fait que** l'étendue en nappe de la deuxième zone (18) est configurée de telle sorte qu'une surface d'appui par laquelle la deuxième zone (18) entre en contact avec la face supérieure du pansement à pression négative laquelle montre dans la direction opposée à la plaie, fait au moins 1,5 fois, en particulier au moins 1,8 fois la taille de la surface d'appui de la première zone (16).

12. Dispositif de raccordement selon l'une ou plusieurs des revendications 9 à 11, **caractérisé par le fait que** l'épaisseur maximale de la première zone (16) du corps d'accouplement (6) surmoulé par injection sur le moyen de conduite (8) est 3 mm tout au plus, en particulier 2 mm tout au plus, plus grande que l'épaisseur du moyen de conduite (4) et que la largeur de la première zone (16) du corps d'accouplement (6) est 5 mm tout au plus, en particulier 3 mm tout au plus, en particulier 2 mm tout au plus, plus grande que la largeur du moyen de conduite (4).

13. Dispositif de raccordement selon l'une ou plusieurs des revendications précédentes, **caractérisé par le fait que** l'étendue en épaisseur du moyen de conduite (4) est de 7 mm tout au plus, en particulier de 6 mm tout au plus, en particulier d'au moins 3 mm, et plus particulièrement comprise entre 4 et 6 mm, la largeur transversale à l'extension longitudinale est d'au moins 12 mm, en particulier d'au moins 15 mm, en particulier comprise entre 15 mm et 30 mm, en particulier comprise entre 15 mm et 25 mm, en particulier entre 15 mm et 22 mm.

14. Dispositif de raccordement selon l'une ou plusieurs des revendications précédentes, **caractérisé par le fait que** le corps d'accouplement (6) comprend au moins trois lumières (36, 38, 40) s'étendant les unes à côtés des autres, une lumière (36) étant réalisée en tant que lumière de rinçage, au moins une lumière étant réalisée en tant que lumière d'aspiration et une lumière formant la lumière destinée à amener un milieu fluide à la plaie, et que la lumière de rinçage et la lumière d'aspiration sont reliées fluidiquement entre elles à leurs extrémités.

15. Dispositif de raccordement selon l'une ou plusieurs des revendications précédentes, **caractérisé par le fait qu'**une face du corps d'accouplement (6) laquelle montre vers le pansement à pression négative est réalisée avec une couche adhésive ou un revêtement adhésif pour établir, pour l'utilisation conforme, une liaison au pansement à pression négative qui est adhésive et pour l'essentiel étanche à la pression négative par rapport à l'environnement.
